(19) Europäisches Patentamt / European Patent Office / Office européen des brevets

(11) **EP 2 411 030 B1**

(12) **EUROPEAN PATENT SPECIFICATION**

(45) Date of publication and mention of the grant of the patent:
**29.01.2014 Bulletin 2014/05**

(21) Application number: **10716619.1**

(22) Date of filing: **26.03.2010**

(51) Int Cl.:
*A61K 36/482* (2006.01)   *A61P 25/22* (2006.01)

(86) International application number:
**PCT/IB2010/000687**

(87) International publication number:
**WO 2010/109318 (30.09.2010 Gazette 2010/39)**

(54) **Herbal extract of Casia tora leaves for use in the treatment of anxiety disorders**

Pflanzenextrakt aus Cassia tora-Blättern zur Behandlung von Angststörungen

Extrait végétal de feuilles de Cassia tora pour traiter des troubles d'anxiété

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **27.03.2009 IN DE06112009**

(43) Date of publication of application:
**01.02.2012 Bulletin 2012/05**

(73) Proprietor: **Council of Scientific & Industrial Research**
**New Delhi 110 001 (IN)**

(72) Inventors:
- **RATHOD, Meena, Rajnikant**
  Gujarat 364 002 (IN)
- **SHETHIA, Bhupendra, Dhanvantrai**
  Gujarat 364 002 (IN)
- **PANDYA jayant, Batukrai**
  Gujarat 364 002 (IN)
- **DODIYA, Prakash, Jagjivanbhai**
  Gujarat 364 002 (IN)
- **PALIT, Gautam**
  Gujarat 364 002 (IN)
- **CHATTERJEE, Manavi**
  Gujarat 364 002 (IN)
- **GUPTA Shibani, Sen**
  Gujarat 364 002 (IN)
- **AGARWAL, Ashok, Kumar**
  Gujarat 364 002 (IN)
- **KHANNA, Vinay, Kumar**
  Gujarat 364 002 (IN)

(74) Representative: **Witte, Weller & Partner**
**Königstrasse 5**
**70173 Stuttgart (DE)**

(56) References cited:
**WO-A1-2007/111401**

- **CHIDUME F C ET AL: "Antinociceptive and smooth muscle contracting activities of the methanolic extract of Cassia tora leaf" MEDICINAL & AROMATIC PLANTS ABSTRACTS, SCIENTIFIC PUBLISHERS, SCIENTIFIC PUBLISHERS, NEW DELHI - INDIA, vol. 24, no. 6, 1 December 2002 (2002-12-01), XP018012159 ISSN: 0250-4367**
- **Abu Bakr Mohamad, Bin Zazakirayya Razi: "Kohl-e- Tarfah" TKDL 1976, XP002591564 Retrieved from the Internet: URL:http://www.tkdl.res.in/tkdl/langdefaul t/common/home.asp [retrieved on 2010-07-12]**
- **Mohammad Najmul Ghani Khan: "Zimaad Barg-e-panwaad" TKDL 1911, XP002591565 Retrieved from the Internet: URL:http://www.tkdl.res.in/tkdl/langdefaul t/common/TKDLSearch.asp?GL=Eng [retrieved on 2010-07-12]**
- **Mohammad Najmul Ghani Khan: "Zimaad Bara-e-Khujaaj-wa-Phunsi" TKDL 1911, XP002591566 Retrieved from the Internet: URL:http://www.tkdl.res.in/tkdl/langdefaul t/common/TKDLSearch.asp?GL=Eng [retrieved on 2010-07-12]**
- **DATABASE WPI Week 200841 Thomson Scientific, London, GB; AN 2008-G47987 XP002591567 & KR 100 772 058 B1 (KIM E Y) 25 October 2007 (2007-10-25)**
- **"Cassia tora" Liber Herbarum II 23 March 2008 (2008-03-23), XP002591568 Retrieved from the Internet: URL:http://web.archive.org/web/ 20080410023 835/http: //www.liberherbarum.com/Pn5608.HT M [retrieved on 2010-07-12]**

- **KINRYS GUSTAVO ET AL: "Natural remedies for anxiety disorders: potential use and clinical applications." DEPRESSION AND ANXIETY 2009 LNKD- PUBMED:19123457, vol. 26, no. 3, 5 January 2009 (2009-01-05), pages 259-265, XP002591569 ISSN: 1520-6394**
- **HEAD KATHLEEN A ET AL: "Nutrients and botanicals for treatment of stress: adrenal fatigue, neurotransmitter imbalance, anxiety, and restless sleep." ALTERNATIVE MEDICINE REVIEW : A JOURNAL OF CLINICAL THERAPEUTIC JUN 2009 LNKD- PUBMED: 19594222, vol. 14, no. 2, June 2009 (2009-06), pages 114-140, XP002591570 ISSN: 1089-5159**
- **YAM MUN FEI ET AL: "An investigation of the anti-inflammatory and analgesic effects of Orthosiphon stamineus leaf extract." JOURNAL OF MEDICINAL FOOD JUN 2008 LNKD- PUBMED: 18598181, vol. 11, no. 2, June 2008 (2008-06), pages 362-368, XP002592678 ISSN: 1557-7600**
- **SOUWALAK PHONGPAICHIT ET AL: "Antifungal activity from leaf extracts of Cassia alata L., CAssia fistula L. and Cassia tora." SONKLANAKARIN J.SCI. TECHNOL, vol. 26, no. 5, 2004, pages 741-748, XP002592679**
- **NEERAJ GILHOTRA ET AL.: "A Review on antianxiety plants" NATURAL PRODUCT RADIANCE, vol. 7, no. 5, 13 February 2008 (2008-02-13), pages 476-483, XP002592680**

**Description**

**FIELD OF THE INVENTION**

The present application relates to a process for the preparation of herbal extract of *Cassia tora* leaves, useful for the treatment of anxiety disorders. The present invention particularly relates to a novel extract of *Cassia tora* leaves, which show anti-anxiety activity. Herein described is a process for the preparation of a novel extract from *Cassia tora* leaves and more particularly the preparation of active extract from leaves of *Cassia tora* plant belonging to *Caesalpiniaceae* family and *Leguminaceae* subfamily. The extract so obtained is a new source of anti- anxiety drug, which can be isolated as defined in the claims and identified. The active extract consists of constituents active against anxiety and useful for the treatment of neurological disease like anxiety.

**BACKGROUND OF THE INVENTION**

Anxiety disorders, the most prevalent psychiatric illness in the general community, are present in 15-20% of medical clinic patients. Anxiety can be defined as an emotional state, unpleasant in nature, uneasiness, discomfort and concern or fear about some defined or undefined future threat or can be a component of, or reaction to, a primary medical disease [Reus V.I. (2005). Mental Disorders. In (16th Edition) Harrison's Principles of internal medicine (pp 2457-2552). Mc-Graw Hill]. Though fear and anxiety are nature's first-line of defense, as the ability to sense potential danger before it strikes is an evolutionary key to survival. But when it is excessive and becomes disproportionate to the situation, and interferes with a person's ability to function effectively, then it leads to clinical anxiety and panic disorders which requires treatment.

The primary anxiety disorders are classified according to their duration and course and the nature of precipitants. Most common of them is the generalized anxiety and the panic disorder. Generalized anxiety disorder is a chronic state of anxiety wherein the patient feels apprehensive, highly alert with an overactive sympathetic system without any reason, whereas panic disorder can be defined by the presence of recurrent and unpredictable panic attacks, which are distinct episode of intense fear and discomfort associated with a variety of physical symptoms, including palpitations, sweating, trembling, shortness of breath, chest pain, dizziness and a fear of impending doom or  trembling, shortness of breath, chest pain, dizziness and a fear of impending doom or death.[Reus V.I. (2005). Mental Disorders. In (16th Edition) Harrison's Principles of internal medicine (pp 2457-2552). Mc-Graw Hill]

Currently, the benzodiazepines are the most commonly employed medicinal treatments for the common clinical anxiety disorders, despite the potential for drug dependence and side effects. Virtually all effects of benzodiazepines results from their depressant actions on central nervous system (CNS). The most prominent of these effects are sedation, hypnosis, anxiolytic, muscle relaxation, anterograde amnesia and anticonvulsant activity. [Charney D.S., Mihic S.J., and Harris R.A. (2006) Hypnotics and Sedatives. In (11th Edition) Goodmann and Gillmann's the pharmacological basis of therapeutics (pp 401-427). Mc-Graw Hill, Baldessarini R.J. (2006) Drug therapy of Depression and Anxiety Disorders. In (11th Edition) Goodmann and Gillmann's the pharmacological basis of therapeutics (pp 429-459). Mc-Graw Hill].

However, at the time of peak concentration in plasma, hypnotic doses of benzodiazepines can be expected to cause varying degrees of side effects including lightheadedness, lassitude, increased reaction time, motor incoordination, impairment of mental and motor functions, confusion and anterograde amnesia. [Charney D.S., Mihic S.J., and Harris R.A. (2006) Hypnotics and Sedatives. In (11th Edition) Goodmann and Gillmann's the pharmacological basis of therapeutics (pp 401-427). Mc-Graw Hill]

Chronic benzodiazepine use poses a risk of development of dependence and abuse [Wood, J.H., Katz, J.L and Winger G. (1992) Benzodiazepines: Use,abuse, and consequences Pharmacol. Rev., 1992, 44:151-347]. After chronic use, the proportion of patients who become tolerant increases, and reducing the dose or stopping the medication produces withdrawal symptoms which may include temporary intensification of the problems that originally prompted their use (e.g., insomnia or anxiety). Dysphoria, irritability, sweating, unpleasant dreams, tremors, anorexia and faintness or dizziness may also occur, especially when withdrawal of the benzodiazepine occurs abruptly [Petursson, H. (1994). The benzodiazepine withdrawal syndrome. Addiction. 89:1455-1459]. Some patients may increase their dose overtime because tolerance definitely develops to the sedative effects. Mild dependency may develop in many patients who have taken therapeutic doses of benzodiazepines on a regular basis for prolonged periods. Abuse by the pregnant mother can result in the  withdrawal symptom of the newborn. [Baldessarini R.J. (2006) Drug therapy of Depression and Anxiety Disorders. In (11th Edition) Goodmann and Gillmann's the pharmacological basis of therapeutics (pp 429-459). Mc-Graw Hill]

Benzodiazepines causes drug interactions by potentiate the effect of other CNS depressants such as alcohol, hypnotics, and neuroleptics. Further aminophyllines antagonizes the action of benzodiazepines and enzyme inhibitors like cimitidine and ketoconazole enhances benzodiazepine actions. Therefore, there is an urgent need of drug which possess a greater efficacy lesser undesirables effect with no tolerance and dependence. [Charney D.S., Mihic S.J., and Harris R.A. (2006) Hypnotics and Sedatives. In (11th Edition) Goodmann and Gilimann's the pharmacological basis of therapeutics (pp 401-427). Mc-Graw Hill]

The use of plants for alleviation of human suffering is perhaps as old as human themselves. Herbal drugs are playing an important role in health care programs and there is resurgence of interest in herbal medicine for treatment of various ailments including neuropsychiatric disorders. Because of their wide biological activities, higher safety margins and lesser costs, herbal medicines are in great demand in the developed as well as developing countries for primary healthcare.

The current Invention relates to the biological efficacy of the leaves part of the plant *Cassia tora* against neurological disorder- anxiety. Anti anxiety activity of the plant has not been reported so far. The crude alcoholic extract of the leaves of the plant revealed significant antianxiety activity. Further, the antianxiety activity was found to be localized in a non-toxic extract of leaves part of the plant *Cassia tora.*

Bhawarlal Jain Babulal in a PCT Patent entitled "A Process for manufacturing an herbal composition for relieving pain from joints and bones" vide Patent No. WO 2008/015697 A3 describes a process for manufacturing an herbal composition comprising of different extracts of various herbs in the different percentage by weight including fruit extract of Cassia tora (15%). The composition is said to be useful for relieving pain from joints and bones. Cited reference does not suggest the use of extract of *Cassia tora* for the treatment of anxiety.

Cherng JM et al, in a research paper entitled "Anthraquinones of edible wild vegetable Cassia tora stimulate proliferation of human CD4(+) T lymphocytes and secretion of interferon - gamma or interleukin 10" published in Food Chemistry, Vol. 107 (4) pp. 1576-1580 (April 2008) describes the immunostimulatory activities of four anthraquinones of Cassia tora on human peripheral blood mononuclear cell (PBMC). The results of the studies showed that at non-cytotoxic concentrations, the tested anthraquinones were effective in stimulating the proliferation of resting human PBMC and /or secretion of IFN-gamma. However, at the concentration of 10 mu gm/ mL (35 M), rhein significantly stimulated proliferation of resting human PBMC (stimulation index (SI) = 1.53), but inhibited IFN-gamma secretion (74.5% of control). Cited reference does not expressly describe the anti-anxiety activity in the Cassia tora plant.

Crane Philip in a PCT Patent entitled "Modified polysaccharides for depressing floatable gangue minerals" vide Patent No. WO 2008/020311 A3 describes a class of non-sulfide, silicate gangue material depressants based on naturally occurring galactomannans having a galactose-to-mannose ratio of less than 1 : 2.1, exemplified by modified products of the seed gums: *Cassia tora* gum, Sesbania gum, Kalonji gum, Ceratonia siliqua gum and/ or *Tamarindus india* gum and methods of making and using them. Cited reference does not teach the usage of alcoholic extract of *Cassia tora* leaves for the treatment of anxiety.

Kim Kwang Soo in a PCT Patent entitled "Beverage composition using Sea weed Fusiforme and onion for the prevention of Hypertension" vide Patent No. WO 2008/032958 A1 describes about the beverage composition for the prevention of Hypertension comprising Hizikia fusiforme, Allium cepa, Acanthopanax senticosus, Lycii fructus, Cassia tora extract and vinegar. It also descloses the preparation of extracts as well their evaluation for anti-hypertensive activity. Cited reference does not expressly describe the anti-anxiety activity in any of the plant species mentioned above except Cassia tora and does not pertain to the present invention.

Qi, Guoyi, in a Chinese patent entitled "Mongolian medicinal composition containing Liquidambar and Terminalia and others for treating psoriasis" vide Patent No. CN 101 167908 A 30 Apr 2008, 8pp. (Chinese) describes the Mongolian medicinal compn. for treating psoriasis composed of Boehmeria nivea seed 35, Cassia seed 35, Liquidambar formosana 35, Terminalia chebula 35, Lotus seed 35, Gardenia jasminoides 35, Xanthoceras sorbifolia 30, Acorus gramineus 30, Trogopterus xanthipes feces 35, crystal salt 35, Viola yedoensis 30, Rhododendron molle 30. Eugenia caryophyllata dried flower bud 30, artificial bezoar 35, buffalo horn powder 35, bear bile 10, Crocus sativus 20, musk 5, baked mercury 10, and baked sulfur 10 wt. part. Cited reference does not teach the anti anxiety activity of Cassia tora leaves. Yan, Liang et al, in a Chinese patent entitled "A Chinese medicinal composition for relieving eye diseases in the middle-aged and senile patients, and preparation method thereof" vide Patent No. CN 101 167954 A 30 Apr 2008, 4pp. (Chinese) describes the preparation of Chinese medicinal compn (by wt. parts) Lycium barbarum fruit 10-25, Rhizoma Polygonati 5-20, Semen Cassiae (Cassia obtusifolia and/or Cassia tora) 10-25, Morus alba fruit 10-25, glue of Equus asinus hide 5-20 and Chrysanthemum morifolium flower 5-20. The prepn. method comprises mixing the water soln. of the fine powder of glue of Equus asinus hide with the concd. water decoction of the rest medicinal materials, adding proper amt. of starch, dextrin and stevioside, granulating with ethanol, drying, sieving and bagging. It has good therapeutic effects on visual deterioration and presbyopia in senile patients. It can improve vision effectively; and has auxiliary functions in relieving asthenopia, protecting the functions of eye tissues, and preventing, relieving and eliminating eye diseases such as myopia, presbyopia and amblyopia. It has no adverse side effect on human bodies. Cited reference does not disclose the anti anxiety activity of Cassia tora leaves.

Li, Shutang, in a Chinese patent entitled "Antihypertensive hypolipidemic healthcare tea" vide Patent No. CN 101120708 A 13 Feb 2008, 7pp. (Chinese) describes the antihypertensive hypolipidemic healthcare tea which comprises (by wt.%): Saliva miltiorrhiza 20-40, Ginkgo biloba leaves 15-35, Gynostemma pentaphyllum 15-35, Semen Cassiae 10-25, Semen Persicae 5-20, and Carthamus tinctorius 5-15. The tea is prepd. by mixing and bagging. The tea can nurse lungs, eyes, livers and kidneys, promote the decompn. of serum cholesterin, triglyceride and low d. lipoprotein, and prevent cardiovascular and cerebrovascular diseases. Cited reference does not disclose the anti anxiety activity of Cassia tora leaves extract.

Wang, Zhihua, in a Chinese patent entitled "Health oral liquid for fat and weight reduction, skin care and gastrointestinal function regulation" vide Patent No. CN 101112457 A 30 Jan 2008,7pp. (Chinese) describes the preparation of health oral liq. from Semen Cassiae (Cassia obtusifolia and/or Cassia tora) 15-25, Radix Et Rhizoma Rhei 15-25, oligosaccharide 3-15, honey 10-15, Cynomorium songaricum 20-30, and tea leaf 2-3%, by decocting Semen Cassiae and Cynomorium songaricum in pure water for 45 min, filtering, soaking tea leaf preferably longjing tea in the filtrate for 30 min, filtering again, cooling the filtrate down to 15, adding the rest of materials, stirring for 10 min, filtering, and bottling. The oral liq. is effective in reducing lipids, cholesterols, triglycerides and low d. lipoproteins in blood, and has anti-arteriosclerotic and antiaging effects. Cited reference does not teach the anti anxiety activity of Cassia tora leaves.

Yang, Rongping et al.,in a Chinese patent entitled "New formulations of traditional Chinese medicine for treating fatty liver" vide Patent No. CN 101112432 A 30 Jan 2008, 19pp. (Chinese) describes the chinese medicinal prepn. for treating primary and secondary fatty liver, which is prepd. from (by wt. parts) Semen Cassiae (Cassia obtusifolia and/or Cassia tora) 9-15, Crataegus pinnatifida 9-15, and Panax quinquefolium 3-9, by reflux-extg, the above materials with 30-90% ethanol soln., removing ethanol, sepg. by macroporous resin column chromatog., eluting with ethanol, collecting ethanol eluate, concg., adding adjuvant, and processing into various pharmaceutically acceptable forms. The invention has the advantages of definite therapeutic effect, low adverse side effect, controllable quality and low cost. Cited reference does not disclose the anti anxiety activity of Cassia tora leaves.

Zheng, Jimi, in a Chinese patent entitled "Chinese medicinal granule for treating retinal hemorrhage and vitreous opacity." vide Patent No. CN 101112468 A 30 Jan 2008,4pp. (Chinese) describes the Chinese medicinal granule preparation for treating retinal hemorrhage and vitreous opacity, which comprises (by wt. parts) Salvia miltiorrhiza 30, Radix Puerariae (Pueraria lobata and/or Pueraria thomsonii) 15, Semen Cassiae (Cassia obtusifolia and/or Cassia tora) 15, Cuscuta chinensis seed 15, Pollen Typhae (Typha angustifolia and/or Typha orientalis) 10, Rhizoma Atractylodis (Atractylodes lancea and/or Atractylodes chinensis) 10, Sargassum (Sargassum pallidum and/or Sargassum fusiforme) 10, Thallus Laminariae and/or Thallus Eckloniae 10, Scutellaria baicalensis 6, and powder of Panax notoginseng 2. The granule is administered orally and is rich in puerarin which is a flavonoid compd. effective in improving microcirculation, enhancing erythrocyte deformability, increasing blood flow rate, and inhibiting platelet aggregation. The granule has the advantages of reasonable formula and marked therapeutic effect. Cited reference docs not suggest the anti anxiety activity of Cassia tora leaves.

Nishikawa, Masashi et al., in Japanese patent entitled "Cassia tora seed extracts, their manufacture, method for reduction of diarrhea-inducing activity of the extracts, and foods and beverages containing them" vide patent No. JP 2008007473 A 17 Jan 2008, 12pp. (Japanese) describes the manufacture of Cassia tora seed exts., useful for prevention and treatment of fatigue, contain (A) emodin, Aloe-emodin, chrysophanol, physcion, aurantioobtusin, and their glycosides, and (B) rubrofusarin (I) or its glycosides in wt. ratio A/B £2.00, preferably £1.90. Cassia occidentalis may be also used instead of C. tora. Thus, 90° water ext. of pulverized C. tora seeds (A/B = 1.619) was orally administered to mice, resulting in causing no diarrhea. Beverage contg. the ext. at 106.7 mg/day as 1 for 3 days raised plasma GSH level from ~1.5 mM to -2.3 mM in healthy volunteers with no adverse effects on digestive functions. Cited reference does not describe the anti anxiety activity of Cassia tora leaves.

Zhang, Y et al, in a research paper entitled "Chemical components and antioxidant activity of the volatile oil from cassia tora L. seed prepared by supercritical fluid extraction" published in Journal Of Food Lipids, Vol. 14 (4) pp. 411-423 (December 2007) describes the extraction of volatile oil from seeds of Cassia tora L. by employing supercritical fluid extraction using carbon dioxide and its analysis by gas chromatography (GC) and GC-mass spectrometry. This volatile oil was characterized by a high content of aliphatic acids (75.90%) and anthraquinones (7.26%). In the antioxidant assays, the volatile oil from C. tora L. seed demonstrated 2,2'-diphenyl-1-picryhydrazyl radical-scavenging activity in the concentration range from 20 to 500 mu g/mL, with the 50% inhibitory concentration (IC50) value at 137 mu g/mL; it also showed a significant inhibitory effect against hydroxyl radicals with an IC50 value of 67 mu g/mL, lower than that of quercetin (IC50 = 8.15 mu g/mL), but superior to that of 4-terpineol (IC50 = 87.5 mu g/mL). The observed antioxidant activity of this volatile oil is probably caused by the presence of high levels of chrysophanol and, possibly, (Z,Z)-9,12-octadecadienoic acid. Cited reference does not pertain to the anti-anxiety activity in the extract of Cassia tora leaves.

Jang DS et al, in a research paper entitled "Anthraquinones from the seeds of Cassia tora with inhibitory activity on protein glycation and aldose reductase" published in Biological & Pharmaceutical Bulletin, Vol. 30 (11) pp. 2207-2210 (November 2007) describes the in-vitro bioassay study of nine anthraquinones isolated from an EtOAc-soluble extract of the seeds of Cassia tora to evaluate their inhibitory activity against advanced glycation end products (AGEs) formation and rat lens aldose reductase (RLAR). Among the isolates, compounds 6 and 8 exhibited a significant inhibitory activity on AGEs formation with observed IC50 values of 118 and 28.9 mu M, respectively, in an AGEs-bovine serum albumin (BSA) assay by specific fluorescence. Furthermore, compounds 6 and 8 inhibited AGEs-BSA formation more effectively than aminoguanidine, an AGEs inhibitor, by indirect AGEs-ELISA. N-epsilon-Carboxymethyllysine (CML)-BSA formation was also inhibited by compounds 6 and 8. Whereas compounds 1, 4, and 6 showed a significant inhibitory activity on RLAR with IC50 values of 13.6, 8.8, and 15.9 mu M, respectively. Cited reference does not teach the anti-anxiety activity in the extract of Cassia tora leaves.

El-Halawany, AM et al, in a research paper entitled "Estrogenic and anti-estrogenic activities of cassia tora phenolic constituents" published in Chemical & Pharmaceutical Bulletin, Vol. 55 (10) pp. 1476-1482 (October 2007) describes an estrogenic activity bioassay-guided fractionation of the 70% ethanolic extract of Cassia tora seeds. Two new phenolic triglucosides, along with seven known compounds were isolated and the structures of the new compounds were elucidated. The estrogenic activity of the fractions and the isolated compounds were investigated using the estrogen-dependent proliferation of MCF-7 cells. In addition, the yeast two hybrid assay expressing estrogen receptor alpha (ER alpha) and beta (ER beta) and the ERa competitor screening assay (ligand binding screen) were used to verify the binding affinities of the isolated compounds to ER. Furthermore, a naringinase pre-treatment of the 70% alcoholic extract of Cassia tora seeds resulted in a significant increase in its estrogenic activity. From the naringinase pre-treated extract six compounds were isolated, among which 6-hydroxymusizin and aurantio-obtusin showed the most potent estrogenic activity, while torachrysone, rubrofusarin and toralactone showed a significant anti-estrogenic activity. Finally, from the structure activity relationship studies it was found that the basic nucleus 1, 3, 8-trihyroxy-naphthalene (T3HN) plays a principal role in the binding affinity of these compounds to ER. Cited reference does not disclose the anti-anxiety activity of the extract from Cassia tora leaves.

Jia, ZB et al, in a research paper entitled "Antioxidant properties of extracts from juemingzi (Cassia tora L.) evaluated in vitro" published in LWT-Food Science And Technology, Vol. 40 (06) pp. 1072-1077 (2007) describes the antioxidant activity of three different fractions of methanol extract from juemingzi (Cassia tora L.) was evaluated using different antioxidant tests, including inhibition of peroxidation of linoleic acid, reducing power, free-radical scavenging and ferrous ions chelating activity. In the above four assays, all the fractions showed antioxidant potential to varying degrees. Among these fractions, ethyl acetate fraction exhibited more antioxidant potency than other fractions. Furthermore, ethyl acetate fraction was found to be more effective in protecting LDL against oxidation in a concentration-dependent manner. The data suggest that juemingzi especially ethyl acetate-soluble fraction may have a preventive effect against atherosclerosis by inhibiting LDL oxidation. Cited reference does not describe the anti-anxiety activity of the extract from Cassia tora leaves.

Cho, IJ et al, in a research paper entitled "Hypolipidemic effect of soluble fiber isolated from seeds of Cassia tora Linn in rats fed a high-cholesterol diet" published in Journal Of Agricultural And Food Chemistry, Vol. 55 (04) pp. 1592-1596 (21 February 2007) describes hypolipidemic effect of soluble fiber isolated from seeds of Cassia tora Linn in rats fed a high-cholesterol diet. In this study the effects of SFC on lipid metabolism were investigated. Male Sprague-Dawley rats were fed one of three experimental diets, a normal diet, a high-cholesterol diet, or a high-cholesterol diet with 5% SFC, for 5 weeks. The serum concentration of total cholesterol in rats fed SFC was 27% lower ($p < 0.05$) compared to that of the control group, but the serum high-density lipoprotein cholesterol level was increased in the SFC group. Liver total cholesterol and triglyceride levels were reduced significantly ($p < 0.05$) in rats fed the SFC diet. In addition, fecal bile acid and lipid excretion was significantly increased by SFC consumption. These results indicate that SFC enhances fecal lipid excretion and may cause a reduction in serum and hepatic lipid concentrations in rats. Cited reference does not expressly describe the anti-anxiety activity of the extract from Cassia tora leaves.

Eladevi Mahendra Shah, in a US Patent entitled "Novel base material for pharmaceutical and/or cosmetic cream (Herbal composition for itchy or infected skin)" vide Patent No. US 2007/0014749 A1 describes the preparation and use of compositions for the treatment of skin disorders itchy and / or infected skin such as impetigo, acne and fungal infection of skin and nails. The compositions are based on extracts from the plants Cassia tora, Centratherum anthelminticum and/or Mclia azadirachta. The herbs may be in powdered form of suitable for preparing decoctions in hot water. Cited reference does not describe the Anti-anxiety activity of the extract of Cassia tora.

Kaneda Yoshihisa et al., in a PCT Patent entitled "Composition having physical endurance improving effect and/or anti-fatigue effect" vide Patent No. WO 2007/004570 A1 describes for a composition comprising a natural product derived component, which comprises an active ingredient different from that in a conventionally known amino acid composition, is effective in the improvement in physical endurance and the alleviation or recovery of the decline in physical activity and fatigue, and is safe for oral administration. A composition having a physical endurance-improving effect and/or an anti-fatigue effect and comprising an active ingredient, a substance extracted from a seed cassiae semen (A seed of a plant Cassia obtusifolia or Cassia tora) with water, an organic solvent compatible with water or a mixture of water with the organic solvent at any given ratio. Cited reference does not expressly teach the Anti-anxiety activity in the extract of Cassia tora leaves.

Nam, Jong Hyun in a PCT Patent entitled "Natural plant extract composition for prevention and recovery of hyperlipidemia and stroke, natural tea containing the same as active ingredient and method for preparing the natural tea" vide Patent No. WO 2007/111401 A1 describes a natural plant extract composition contains a Cassia tora extract and an Albizzia julibrissin extract which can lower the blood cholesterol level to prevent arteriosclerosis and stroke. And it is effective for the prevention and treatment of hyperlipidemia and stroke. Cited reference does not expressly describe the Anti-anxiety activity of the extract of Cassia tora leaves.

Dutta, Tuhina et al, in a research paper entitled "Identification of antifungal activity in different species of Cassia" published in Indian Journal of Environment and Ecoplanning, 12(3), 661-663 (English) 2006 describes the antifungal activity in three different species of Cassia viz. Cassia fistula, Cassia sophera and Cassia tora. The crude 50% aq. ethanolic leaf ext. of the three species were tested for their antifungal effect by the std. Agar cup method. Antifungal activity was located in the crude exts. of all the tested plant exts. against Fusarium oxysporum and Phytophthora sp. The antifungal property was also evaluated by studying the effects of the leaf exts. on healthy and fungal infected seeds of Phaseolus aureus in different doses (2mg/mL, 2.4mg/mL, 2.8mg/mL and 3.2mg/mL). Maximum inhibitory effect was exhibited by Cassia sophera (3.2mg/mL dose). Cited reference does not expressly describe the anti-anxiety activity of the extract from Cassia tora leaves.

Guo Xinfu, in a PCT Patent entitled "A composition for treating or preventing hyperlipemia, hypertension and hepatic adipose infiltration" vide Patent No. WO 2006/081739 A1 describes the preparation of a composition for treating or preventing hyperlipemia, hypertension and hepatic adipose infiltration. The composition contains various herbs including semen of Cassia tora L. which has no toxic side effect and have good effects on regulating fats, can significantly reduce cholesterol, triglyceride, low density lipo-protein in the blood, have apparent effects on treating and preventing hypertension, hepatic adipose infiltration, and can be used in preparing drugs or health food for treating and preventing hyperlipemia, hypertension and hepatic adipose infiltration Cited reference does not suggest the anti-anxiety activity in the extract of Cassia tora leaves.

Kim S. Y. et al in a Korean Patent entitled "Composition for preventing and treating anxiety symptom associated with brain nervous system comprising extract of cinnamomum cassia presl. Having anti-anxiety activity" vide Patent No. KR2006030535-A; KR646594-B1, describes the preparation of extract of cinnamomum cassia presl. Using a polar solvent selected from water, C1-C4 lower alcohol and their mixed solvent for preventing and treating anxiety symptom associated with brain nervous system. Cited reference does not disclose *in-vitro & in-vivo* antianxiety activity of extract obtained from *Cassia tora* of *Caesalpiniaceae* family. Cho, SH et al, in a research paper entitled "Effects of Cassia tora fiber supplement on serum lipids in Korean diabetic patients" published in Journal Of Medicinal Food, Vol. 8 (3) pp. 31 1-318 (2005) describes the effects of Cassia tora fiber supplement on serum lipids in Korean diabetic patients. Cassia tora fiber supplement consisting of 2 g of soluble fiber extracted from Cassia semen (C. tora L.), 200 mg of a-tocopherol, 500 mg of ascorbic acid, and 300 mg of maltodextrin was formulated in a pack, and given to 15 type II diabetic patients with instructions to take two packs per day for 2 months. Lifestyle factors and dietary intakes of the patients were not altered during the 2-month period. Serum total cholesterol was moderately (P<.1) decreased in the C. tora group compared with the age- and gender-matched placebo group, as was the ratio of apolipoprotein B to apolipoprotein A 1 (P < . 1). Levels of serum triolycerides and low-density lipoprotein-cholesterol tended to decrease more in the C. tora-supplemented group than in the placebo group. Serum a-tocopherol was increased (P<.01) but lipid peroxides were not significantly lower in the C. tora group. Fasting blood glucose, hemoglobin A 1 c, blood urea nitrogen, creatinine, and activities of serum aspartatc aminotransferase and alanine aminotransferase were not changed by the fiber supplement. It was concluded that C. tora supplements can help to improve serum lipid status in type II diabetic patients without serious adverse effects.Cited reference does not teach the anti-anxiety activity of the extract from Cassia tora leaves.

Lee,CH & Lee, HS, in a research paper entitled "antifungal property of dihydroxyanthraquinones against phytopathogenic fungi" published in Journal Of Microbiology And Biotechnology, Vol. 15 (2) pp. 442 - 446 (April 2005) describes the fungicidal activities of Cassia obtusifolia extracts and their active principles against Botrytis cinerea, Erysiphe graminis, Phytophthora infestans, Puccinia recondita, Pyricularia grisea, and Rhizoctonia solani, and compared with synthetic fungicides and two dihydroxyanthraquinones. Cited reference does not describe the anti-anxiety activity of the extract from Cassia tora leaves.

Utz, Ferdinand et al., in a PCT Patent and US Patent entitled "Cationic Cassia derivatives and applications therefor" vide Patent No. WO 2004/1 12733 A 1 and US 2008/0004340 A I US 7,262,157 B2 describes the cationically derivatized polygalactomannans obtained from Cassia tora and Cassia obtusifolia and their use in personal care, household care and institutional care composition. In addition, these polymers are useful for improving the psychosensory and aesthetic properties of cosmetic formulations. Cited reference does not expressly teach the Anti-anxiety activity of the extract of Cassia tora leaves.

Chi-Hao Wu and Gow-Chin Yen, in a research paper entitled "Antigenotoxic properties of Cassia tea (Cassia tora L.): Mechanism of action and the influence of roasting process" published in Life Sciences, Vol. 76 (1) pp. 85 - 101 (November 2004) describes the Antigenotoxic properties and the possible mechanisms of water extracts from *Cassia tora* L. (WECT) treated with different degrees of roasting (unroasted and roasted at 150 and 250°C). The individual anthraquinone content in extracts of *C. tora* was measured by HPLC. Three anthraquinones, chrysophanol, emodin and rhein, have been detected under experimental conditions. The anthraquinone content decreased with increased roasting temperature. In conclusion, our data suggest that the decrease in antigenotoxic potency of roasted *C. tora* was related to the reduction in their anthraquinones. Cited reference does not disclose the anti-anxiety activity of the extract from Cassia tora leaves.

Kim, YM et al., in a research paper entitled "Anthraquinones isolated from Cassia tora (Leguminosae) seed show an antifungal property against phytopathogenic fungi" published in Journal Of Agricultural And Food Chemistry, Vol. 52 (20) pp. 6096 - 6100 (6 October 2004) describes the fungicidal activities of Cassia tora extracts and their active principles against Botrytis cineria, Erysiphe graminis, Phytophthora infestans, Puccinia recondita, Pyricularia grisea, and Rhizoctonia solani using a whole plant method in vivo and compared with synthetic fungicides and three commercially available anthraquinones. Little or no activity was observed for anthraquinone and anthraquinone-2-carboxylic acid when tested at 1 g/L. Chlorothalonil and dichlofluanid as synthetic fungicides were active against P. infestans and B. cinerea at 0.05 g/L, respectively. The results demonstrate the fungicidal actions of emodin, physcion, and rhein from C. tora. Cited reference does not teach the anti-anxiety activity of the extract from Cassia tora leaves.

Hebbar, SS et al., in a review article entitled "Ethnomedicine of Dharwad district in Karnataka, India - plants used in oral health care" published in Journal Of Ethnopharmacology, Vol. 94 (2-3) pp. 261-266 (October 2004) describes the ethnomedicine survey of the Dharwad district of Karnataka in southern India. It was revealed that 35 plants belonging to 26 families are being used to treat different types of oral ailments like toothache, plaque and caries, pyorrhea and aphthae. Sixteen of these plants were new claims for the treatment of oral ailments not previously reported in the ethnomedicinal literature of India. Basella alba, Blepharis repens, Capparis sepiaria, Oxalis corniculata and Ricinus communis are used for the treatment of aphthae; Azima tetracantha, Caesalpinia coriaria, Cleome gynandra, Gossypium herbacium, Leucas aspera, Merremia chryseides, Pergularia daemia, Prosopis juliflora and Solanum nigrum are used to treat tooth ache and Cassia hirsuta and Cassia tora are used in the treatment of plaque and caries. Cited reference does not pertain to the anti-anxiety activity of the extract from Cassia tora leaves.

Park, TH et al., in a research paper entitled "Peroxynitrite scavenging mode of alaternin isolated from Cassia tora" published in Journal Of Pharmacy And Pharmacology, Vol. 56 (10) pp. 1315 - 1321 (October 2004) describes a potent oxidant Peroxynitrite (ONOO) which contributes to the oxidation of various cellular constituents, including lipids, amino acids, sulfhydryls and nucleotides. It can cause cellular injury, such as DNA fragmentation and apoptotic cell death. ONOO- toxicity is also reported to be involved in inflammatory and neurodegenerative diseases, such as Alzheimer's disease, Parkinson's disease and atherosclerosis. Moreover, the necessity for a strong ONOO-scavenger is important because of the lack of endogenous enzymes that protect against the damage caused by ONOO-. The aim of the study was to evaluate the ability of natural products to scavenge ONOO-. They have tested various plant extracts for their ONOO- scavenging activity. Among them, extract from Cassia tora showed potent ONOO- scavenging activity. Further analysis identified the phenolic active components, alaternin and nor-rubrofusarin glucose, as potent ONOO-scavengers. The alaternin and nor-rubrofusarin glucose led to a decrease in the ONOO--mediated nitration of tyrosine through electron donation. In bovine serum albumin, alaternin, but not nor-rubrofusarin glucose, showed significant inhibition of ONOO--mediated nitration in a dose-dependent manner. Therefore, it was assumed that alaternin can be developed as an effective ONOO- scavenger for the prevention of ONOO--associated diseases. Cited reference does not disclose the anti-anxiety activity of the extract from Cassia tora leaves.

Patil, UK et al., in a research paper entitled "Hypolipidemic activity of seeds of Cassia tora Linn" published in Journal Of Ethnopharmacology, Vol. 90 (2-3) pp. 249 - 252 (February 2004) describes the hypolipidemic activity of ethanolic extract of seeds of Cassia tora L. and its fractions on triton induced hyper-lipidemic profile. Ethanolic extract and its ether soluble and water soluble fraction decreased serum level of total cholesterol on the other hand increased the serum HDL-cholesterol level. Ethanolic extract, ether fraction and water fraction decreased triglyceride level and reduced the LDL-cholesterol level. Cited reference does not expressly describe the anti-anxiety activity of the extract from Cassia tora leaves.

Lee, HS. in a research paper entitled "Inhibitory effects of quinizarin isolated from Cassia tora seeds against human intestinal bacteria and Aflatoxin B-1 biotransformation" published in Journal Of Microbiology And Biotechnology, Vol. 13 (4) pp. 529 - 536 (August 2003) describes the growth-inhibitory activity in vitro of Cassia tora seed-derived materials against seven intestinal bacteria and compared with that of anthraquinone, anthraflavine, anthrarufin and 1-hydroxyanthraquinone. The active constituent of C. tora seeds was characterized as quinizarin. The growth responses varied depending on the compound, dose, and bacterial strain tested. At I mg/disk, quinizarin exhibited a strong inhibition of Clostridium perfringens and moderate inhibition of Staphylococcus aureus without any adverse effects on the growth of Bifidobacterium adolescentis, B. bifidum, B. longum, and Lactobacillus casei. Furthermore, the isolate at 0.1 mg/disk showed moderate and no activity against C. perfringens and S. aureus. The structure-activity relationship revealed that anthrarufin, anthraflavine, and quinizarin moderately inhibited the growth of S. aureus. However, anthraquinone and 1-hydroxyanthraquinone did not inhibit the human intestinal bacteria tested. As for the morphological effect of 1 mg/disk quinizarin. most strains of C. perfringens were damaged and disappeared. indicating that the strong activity of quinizarin was morphologically exhibited against C. perfringens. The inhibitory effect on aflatoxin B-1 biotransformation by anthraquinones revealed that anthrarufm (IC50, 11.49 muM), anthraflavine (IC50, 26.94muM), and quinizarin (IC50, 4.12 muM) were potent inhibitors of aflatoxin B-1-8,9-epoxide formation. However, anthraquinone and 1-hydroxyanthraquinone did not inhibit the mouse liver microsomal sample to convert aflatoxin B, to aflatoxin B,-8,9-epoxide. These results indicate that the two hydroxyl groups on A ring of anthraquinones may be essential for inhibiting the formation of aflatoxin B-1-8,9-epoxide. Accordingly, as naturally occurring inhibitory agents, the C. tora seed-derived materials could be useful as a preventive agent against diseases caused by harmful intestinal bacteria, such as clostridia, and as an inhibitory agent for the mouse liver microsomal conversion of aflatoxin B- to aflatoxin B-1-8,9-epoxide. Cited reference does not disclose the anti-anxiety activity of the extract from Cassia tora leaves.

Takagi Chiyoshi et al., in a PCT and EP Patents entitled "Flavor deterioration inhibitor and inhibitor for the generation of citral deterioration smell" vide Patent No. WO 03/105599 A1, EP 01554938 B1 & EP 01554938 Aldescribes for a flavor deterioration inhibitor which comprises an extract obtained by extracting Angelica keiskei, avocado, Cassia tora, Plantago asiatica L, hawthorn, fermented tea leaves with water, an organic polar solvent or a mixture thereof; and a deterioration smell inhibitor for citral or a citral-containing product. In particular, a remarkable inhibitory effect can be achieved on deterioration due to light. Cited reference does not describe the Anti-anxiety activity in the extract of Cassia tora leaves.

Chidume, FC et al., in a research publication entitled "Antinociceptive and smooth muscle contracting activities of the methanolic extract of Cassia tora leaf" published in Journal Of Ethnopharmacology, Vol. 81 (2) pp. 205-209 (July 2002) describes the antinociceptive and smooth muscle contracting activities of the methanolic extract of Cassia tora leaf. The leaves of Cassia tora Linn. (Family: Caesalpiniaceae) were soxhlet extracted with methanol. The spasmogenic effects of the extract were evaluated on guinea pig ileum, rabbit jejunum and mice intestinal transit. Antinociceptive activity of the extract was also evaluated in the mice. The LD50 values of the extract in mice were > 2000 mg/kg i.p. and p.o. The extract contracted smooth muscles of guinea pig ileum and rabbit jejunum in a concentration-dependent manner. Atropine reversibly blocked this activity. Mepyramine also reduced the contractile amplitude due to the extract in a concentration-dependent manner. The extract increased intestinal transit in mice dose dependently. C. tora extract significantly (P < 0.05) reduced the number of acetic acid induced abdominal constrictions in mice and the effect was comparable to that of aspirin (150 mg/kg i.p.). The extract also significantly (P < 0.05) reduced the nociceptive response of mice to increased force (g). The effects were dose-dependent. The studies suggest that the use of C. tora, traditionally, as a purgative and in the treatment of other ailments is justifiable. Cited reference does not expressly teach the anti-anxiety activity of methanolic extract of Cassia tora leaves at ambient temperature. Yen, GC et al., in a research publication entitled "Antioxidant properties of water extracts from Cassia tora L. in relation to the degree of roasting" published in Journal Of Agricultural And Food Chemistry, Vol. 48 (7) pp. 2760-2765 (July 2000) describes the antioxidant properties of water extracts from Cassia tora L. in relation to the degree of roasting. The antioxidant properties of water extracts from Cassia tora L. (WECT) prepared under different degrees of roasting were investigated. The water extracts of unroasted C. tora L. (WEUCT) showed 94% inhibition of peroxidation of linoleic acid at a dose of 0.2 mg/mL, which was higher than that of alpha-tocopherol (82%). Water extracts prepared from C. tora L. roasted at 175 degrees C for 5 min and at 200 degrees C for 5 min exhibited 83% and 2%, respectively, inhibition of linoleic acid peroxidation. This result indicated that the antioxidant activities of WECT decreased with longer roasting time or higher roasting temperature. In conclusion, the decrease in the antioxidant activity of water extracts from roasted C. tora L. might have been due to the degradation of Maillard reaction products and the decrease of polyphenolic compounds. Cited reference does not disclose the anti-anxiety activity of Cassia tora leaves.

El-Tahir, A et al., in a research publication entitled "Antiplasmodial activity of selected sudanese medicinal plants with emphasis on Acacia nilotica" published in Phytotherapy Research, Vol. 13 (6) pp. 474 - 478 (September 1999) describes the antiplasmodial activity of selected sudanese medicinal plants with emphasis on Acacia nilotica. Twenty-two plant organs from eleven plants comprising five families were extracted and screened for antiplasmodial activity in vitro against Plasmodium falciparum 3D7 (chloroquine sensitive) and Dd2 (chloroquine resistant and pyrimethamine sensitive). Fifty nine percent of plant extracts from 22 extracts exerted activity on P. falciparum strain 3D7 with an IC50 less than 50 mu g/mL, whereas 43% of plant extracts showed an IC50 value within 50 mu g/mL on Dd2 strains. Plant extracts from Gardenia lutea, Haplophyllum tuberculatum, Cassia tora, Acacia nilotica and Aristolochia bracteolata possessed IC50 values less than 5 mu g/mL on both tested strains, Bioassay guided fractionation of A. nilotica revealed that the ethyl acetate extract possessed the highest activity (IC50 = 1.5 mu g/mL). Fraction 2 (R-f = 0.75) prepared by preparative chromatography showed the highest activity on P, falciparum (IC50 = 1.7 mu g/mL). Phytochemical analysis indicated that the most active phase contained terpenoids and tannins and was devoid of alkaloids and saponins, The effect of plant extracts on lymphocyte proliferation showed low toxicity to the hunan cells. This plant has been subjected to long term clinical trials in folk medicine and is a promising plant. Cited reference does not expressly describe the anti-anxiety activity of Cassia tora leaves. Hatano, T et al., in a research publication entitled "Phenolic constituents of cassia seeds and antibacterial effect of some naphthalenes and anthraquinones on methicillin-resistant Staphylococcus aureus" published in Chemical & Pharmaceutical Bulletin, Vol. 47 (8) pp. 1121 - 1127 (August 1999) describes the isolation of thirteen phenolic glycosides including six new compounds from seeds of Cassia tora (Leguminosae). The effects of the phenolic glycosides, their aglycones and several other compounds structurally related to them on Escherichia coli K12, Pseudomonas aeruginosa PAOI and some strains of Staphylococcus aureus were examined. Among them, torachrysone (15), toralactone (16), aloe-emodin (18), rhein (19) and emodin (20) showed noticeable antibacterial effects on four strains of methicillin-resistant Staphylococcus aureus with a minimum inhibitory concentration of 2-64 mu g/ml. On the other hand, the phenolic compounds tested did not show strong antibacterial effects on E. coli and P. aeruginosa. Cited reference does not suggest the anti-anxiety activity of Cassia tora leaves. Agarkar, SV et al., in a review article entitled "Photochemical and pharmaco-logical investigations of genus Cassia: A review" published in Asial Journal Of Chemistry, Vol. 11 (2) pp. 295 - 299 (April-June 1999) describes the phytochemical and pharmacological investigations of genus Cassia. About 26 species of genus Cassia have been reported to contain anthracene derivatives. The more important species among them are Cassia alata, Cassia angustifolia vahl, Cassia autifolia, Cassia cinnamon, Cassia fistula Cassia nodosa Cassia obtusifolia, Cassia sophera, Cassia tora and Cassia zeylanicum. Diuretic, antidiabetic, antibiotic, antifungal, antipyretic, anthlemintic, antibacterial, antirheumatic, anti-eczema, antiherpetic, antiasthmatic, anti-leprosy, anti-typhoid and anti-bronchitis activities have been reported from some of these species. Cited reference does not suggest the anti-anxiety activity of Cassia tora leaves

Maity, TK et al., in a research paper entitled "Studies on antiinflammatory effect of Casia tora leaf extract (fam. Leguminosae)" published in Phytotherapy Research, Vol. 12 (3) pp. 221 - 223 (May 1998) describes the antiinflammatory effect of the methanol extract of the leaves of Cassia tora against carrageenin, histamine, serotonin and dextran-induced rat hind paw oedema, It exhibited significant antiinflammatory activity against all these agents, The extract (400 mg/kg) showed maximum inhibition of oedema of 40.33%, 31.37%, 53.57% and 29.15% at the end of 3 h with carrageenin, dextran, histamine and serotonin-induced rat paw oedema, respectively. Using a chronic test, the granuloma pouch in rats, the extract exhibited a 48.13% reduction in granuloma weight. Cited reference does not disclose the anti-anxiety activity of methanolic extract of Cassia tora leaves.

Yen, GC et al., in a research paper entitled "Extraction and identification of an antioxidative component from Jue Ming Zi (Cassia tora L.)" published in Journal Of Agricultural And Food Chemistry, Vol. 46 (3) pp. 820 - 824 (March 1998) describes the antioxidant activity of Cassia tora L. and Cassia occidentalis L. The results indicated that methanolic extracts from C. tora L. (MECT) and C. occidentalis L. (MECO) produced stronger antioxidant activity and gave higher yields of extract than other organic solvents, The MECT showed stronger antioxidant activity than did the MECO on peroxidation of linoleic acid. MECT at 200 ppm was stronger than 200 ppm of alpha-tocopherol, but weaker than 200 ppm of butylated hydroxyanisole. Amberlite XAD-2 column chromatography separated MECT into eight fractions. Of the eight fractions, fraction V possessed significant antioxidant activity and showed 85.8% inhibition on peroxidation of linoleic acid. Subsequently, fraction V was separated into two subfractions, Va and Vb, by Toyoperal HW-40 F gel filtration chromatography. The subfraction Vb exhibited stronger antioxidant activity than did subfraction Va and was identified as 1, 3, 8-trihydroxy -6-methyl-9,10-anthracenedione (emodin). Cited reference does not expressly describe the anti-anxiety activity of methanolic extract of Cassia tora leaves.

Pandey, VN et al., in a research paper entitled "Synergistic activity of extracted plant oils against Pythium aphanidermatum and P-debaryanum" published in Tropical Agriculture, Vol. 74 (2) pp. 164 - 167 (April 1997) describes the synergistic activity of extracted plant oils against Pythium aphanidermatum and P-debaryanum. The leaves of Cassia tora, Hyptis suaveolens, Murraya koenigii, Ocimum canum, and Ranunculus arvensis showed absolute volatile toxicity against Pythium aphanidermatum and P. debaryanum. The volatile antifungal fractions of H. suaveolens, M. koenigii, and O. canum were isolated in the form of essential oils. The oils were tested individually as well as in four mixtures. The fungitoxic activity of the four mixtures of the three oils was found to be several times enhanced than that of the individual oils except for O. canum which exhibited absolute fungitoxicity at 1000 ppm. Cited reference does not disclose the anti-anxiety activity of methanolic extract of Cassia tora leaves.

Choi, JS et al., in a research paper entitled "In vitro antimutagenic effects of anthraquinone aglycones and naphthopyrone glycosides from Cassia tora" published in Planta Medica, Vol. 63 (1) pp. 11 - 14 (February 1997) describes the antimutagenic activity of a methanol extract of Cassia tora seeds against aflatoxin B-1(AFB(1)) with the Salmonello typhimurium assay. The MeOH extract was then sequentially partitioned with CH2Cl2. n-BuOH and H2O. The CH2Cl2 and n-BuOH fractions possessed antimutagenic activity but the H2O fraction was inactive. Neither the MeOH extract nor its fractions were capable of inhibiting the direct-acting mutagen N-methyl-N'-nitro-N-nitrosoguanidine suggesting that these fractions may prevent the metabolic activation of AFB(1) or scavenge the electrophilic intermediate capable of inducing mutations. The pure chrysophanol, chryso-obtusin, and aurantio-obtusin obtained from the CH2Cl2 fraction and cassiaside and rubro-fusarin gentiobioside obtained from the n-BuOH fraction demonstrated significant antimutagenic activity. Cited reference does not suggest the anti-anxiety activity of methanolic extract of Cassia tora leaves.

Mukherjee, PK et al., in a research paper entitled "Antifungal activities of the leaf extract of Cassia tora Linn (Fam Leguminosae)" published in Phytotherapy Research, Vol. 10 (6) pp. 521-522. (September 1996) describes the antifungal activities of the leaf extract of Cassia tora Linn. The antifungal activity of the dealcoholized extract of leaves of Cassia tora L, was determined on five different fungal organisms. The crude leaf extract significantly inhibited the growth of C. albicans, A. niger, S. cerevisiae and T. mentagophytes when tested by turbidity and spore germination methods in a concentration dependent fashion. The effects produced by the extract were compared with a standard antifungal agent griseofulvin. Cited reference does not teach the anti-anxiety activity of methanolic extract of Cassia tora leaves.

Sui-Ming Wong et al., in a research paper entitled "New Antihepatotoxic Naphthopyrone Glycosides from the Seeds of Cassia tora1" published in Planta Medica, Vol. 55 (3) pp. 276-280. (June 1989) describes the isolation of new antihepatotoxic naphthopyrone glycosides from the seeds of *Cassia tora.* Their structures were elucidated on the basis of chemical and spectral data. The naphtho-γ-pyrone glycosides were found to have significant hepato-protective effects against galactosamine damage, which were higher than that of silybin from *Silybum marianum.* Cited reference does not disclose the anti-anxiety activity of Cassia tora leaves.

Sui-Ming Wong et al., in a research paper entitled "Anthraquinone glycosides from the seeds of Cassia tora" published in Phytochemistry, Vol. 28 (1) pp. 211-214. (1989) describes the isolation of three new anthraquinone glycosides from the seeds of *Cassia tora.* Their structures were elucidated on the basis of chemical and spectral data. The first two compounds exhibited a weak protective effect on primary cultured hepatocytes against carbon tetrachloride toxicity. Cited reference does not suggest the anti-anxiety activity of *Cassia tora* leaves.

Bayerlein et al., in a US Patent entitled "Derivatives of Cassia tora polysaccharides and their use" vide Patent No. 4,753,659 dated June 28, 1988 describes about new alkyl ethers and phosphoric acid esters of Cassia tora polygalactomannans and their use, alone or in combination with other thickening agents, as thickening agents. Cited reference does not describe the Anti-anxiety activity or any type of activity in *Cassia tora*.

Park H.Y. in a Korean Patent entitled "Eye bandage for sleeping which is made out of fabric dyed by loess, and filled with cassia tora and siberian chrysanthemum, etc" vide  Patent No. KR712055-B1 describes the eye bandage for sleeping comprises: a bandage cover, made by sewing a fabric dyed by the loess through the immersion in the loess solution in such a manner that it is filled with fillers, and having an eye covering member which has a length enough to cover user's both eyes, and an ear covering member which is formed respectively in both sides of the eye covering member; and a filler, composed of the Cassia tora which is filled into the eye covering member. Cited reference does not teach about the antianxiety activity of extract obtained from the *Cassia tora.*

## OBJECT OF THE INVENTION:

The main object of the present invention is to provide a plant extract as defined in claims 1 to 4 for use in treating anxiety disorders, which obviates the drawbacks as detailed above.

Herein described is an active extract from a herbal source.

Herein described is the preparation of a *Cassia tora* leaves extract in solvent mixture having different polarity for antianxiety activity.

In the present application is shown that the leaves of *Cassia tora* plant species possess antianxiety activity.

Herein described is the utilization of a alcohol: water combination for complete extraction.

The present application shows that the plant material retains the activity when stored under ambient condition and the extract prepared from the leaves has high shelf life without loss of activity.

The extract may be used for biological screening, both *in-vitro* and *in-vivo*.

Still another object of the present invention is to provide an antianxiety extract of *Cassia tora* leaves.

## SUMMARY OF THE INVENTION:

Accordingly the present invention provides a process for the preparation of herbal extract of *Cassia tora* leaves, for use in the treatment of anxiety disorders, wherein the extract is prepared by a process comprising the steps of:

> i. collecting *Cassia tora* plant material;
> ii. drying the plant material at ambient temperature in the range of 25 to 37°C while maintaining the moisture in the range of 0.5-1.5 percent;
> iii. pulverizing the plant material dried in step (ii) and selecting the material in the size range of 16 - 20 mesh sieve;
> iv. soaking the pulverized plant material obtained in (iii) in 95:05 (v/v) alcohol-water mixture in static condition at ambient temperature followed by draining & collecting the extract solution;
> v. repeating the soaking step described in step (iv) above five times with fresh solvent mixture at an interval of 24 hours;
> vi. concentrating the extract solution obtained in step (v) by known methods;
> vii. freeze drying the concentrated extract obtained in step (vi) at a temperature in the range of -50 to -60°C for a period in the range of 8 to 16 hours;
> viii. testing the extract *in-vitro* and *in-vivo* for anti-anxiety activities.

In an embodiment of the invention, the plant part used for extraction is leaves of *Cassia tora*.

In another embodiment of the invention, the said extract is active on benzodiazepine receptors.

In another embodiment of the invention, the extract inhibits benzodiazepine receptors even at concentration as low as 1.56 μg.

In yet an embodiment of the invention, the extract is administered in a form selected from tablets, lozenges, capsules, powder, solution, intravenously and orally.

## DETAILED DESCRIPTION OF THE INVENTION:

The plant was identified and required material was collected from the natural vegetation. The extraneous matter was removed from the collected material. The cleaned plant material was dried at room temperature for a period of 3 to 4 weeks. The plant material was pulverized and sieved to obtain 16 to 20-mesh size powder and then soaked in methanol-water mixture under static condition at ambient temperature. The extraction cycle was repeated for five times using fresh alcohol: water (95:05 v/v) mixture. Each extract filtrate was combined, and concentrated using rotary evaporator to obtain crude concentrated extract. The concentrated extract was dispersed in deionised water and again concentrated on rotary evaporator to obtain an extract, free from traces of alcohol. This alcohol-free concentrated extract was freeze dried to obtain moisture-free crude extract in powder form. The crude extract was initially tested *in-vitro* and after positive test the extract was tested for in-vivo anti-anxiety activity.

*In vitro* radioligand receptor binding to assess the effect of extract on frontocortical benzodiazepine receptors:

Role of benzodiazepine receptors in anxiety is well documented. Keeping in view of this, potential of *Cassia tora* leaves extract on frontocortical benzodiazepine receptors was assessed involving *in vitro* assays of 3H-Flunitrazepam to rat frontocortical membranes, known to label benzodiazepine receptors.

## Preparation of crude synaptic membrane:

Albino rats of Wistar strain obtained from the animal breeding colony of Indian Institute of Toxicology Research (IITR), Lucknow were used through out these studies. Rats were sacrificed and their brain removed immediately. The brain was dissected into regions to take out frontal cortex following the standard protocol (Glowinski and Iverson 1966). Crude synaptic membrane from frontal cortex was prepared (Khanna et al 1994). Briefly, frontal cortex was weighed and homogenized in 19 vols. of TRIS - HCl buffer (5 mM, pH 7.4). The homogenate was centrifuged at 50,000 X g for 20 minutes at 4 °C. The supernatant was removed and the pellet was suspended in the same buffer followed by centrifugation at 50,000 X g for 20 minutes at 4 °C to remove endogenous amines and for cell lysis. The pellet thus obtained was finally suspended in the same volume of TRIS - HCl buffer (40 mM, pH 7.4) and used for benzodiazepine receptor assay. Protein estimation was carried out following the procedure of Lowry et al (1951) using bovine serum albumin as a reference standard.

## Radioligand receptor Binding assay:

Assay for benzodiazepine receptors was carried out in 96 well multiscreen plates HTS FB (Millipore, USA) using 4 tip robotic liquid handling system (Multi Probe II EX, Perkin Elmer, USA). Assay mixture in a final volume of 250 ul contained TRIS - HCl buffer (40 mM, pH 7.4, 140 ul), fronto - cortical membrane (50 ul), radioligand (3H - Flunitrazepam, $0.5 \times 10^{-9}$ M, Specific Activity 82 Ci / mmole, Perkin Elmer, USA, 40 ul) and *Cassia tora* leaves extract, concentrations ranging from 1.56 - 200 ug in 20 ul) in multiwell plates in triplicate. To assess the total binding, the assay mixture did not contain test compound and thus volume of the TRIS - HCl buffer was 160 ul. In parallel assay, the reaction mixture contained diazepam ($1 \times 10^{-6}$ M) instead of test compound. Diazepam in that case served as a positive competitor. The contents in the plate were mixed thoroughly and incubated for 15 minutes at 37 °C using plate incubator (Heidolph, Titramax 100). Following incubation, the contents of the plates were filtered over vacuum manifold attached with the robotic liquid handling system. The plates were washed twice with cold TRIS- HCl buffer (40 mM, pH 7.4, 250 ul) to remove unbound radioligand. The plates were dried over vacuum and left on the deck overnight. Following this, scintillation fluid (Microscint O, 60 ul, Perkin Elmer, USA) was added to each well. The plates were stabilized by leaving them overnight and counted on plate counter (TOPCOUNT NXT, Perkin Elmer, USA) for tritium.

Percent inhibition of receptor binding was calculated in presence and absence of extract by the following formulae, 1

$$\% \text{ Inhibition in binding} = \frac{\text{Total binding} - \text{Binding in presence of leaves extract}}{\text{Binding in absence of leaves extract}} \times 100$$

**Material and method for *In vivo* evaluation of the extract:**

**Materials:**

**1. Animals:** Swiss Albino male mice 20-25 gm body weights were procured from Laboratory Animal Services Division of Central Drug Research Institute, Lucknow. All animals were kept in polyacrylic cage in environmentally controlled rooms (temperature 24-27°C and humidity 60-65% with 12:12 light: dark cycle) .Food was provided in the form of dry pellets and water *ad libitum*. Each animal was used only once in the behavioral tests and were habituated with the experimental room one day prior to the experiment All experiments involving animals complies with the ethical standards of animal handling and approved by Institutional Animal Ethics Committee.
**2. Drugs:** Diazepam, [Ranbaxy, India] as a positive control.

**Methods:**

**Treatment of animals:** Diazepam was diluted in distilled water, administered by intraperitoneally. The extract was dissolved in 0.5% gum acacia suspension and administered by oral route.
**Elevated plus maze test in mice:** This test was used to evaluate the anti-anxiety activity. The test is based on the principal of conflict between exploration and fear of open/ or closed arms. Apparatus consists of a raised stainless steel maze of four arms arranged in '+' sign. Two of opposite arms are enclosed by raised walls whereas other arms are kept open. All the four arms consist of infrared beams at regular distance. The movement of animal across the arms is calculated by interruption of beams, which was analyzed by Maze Tracking Software (M/s. Columbus Instruments, USA). The trial was started by placing an animal on the central platform of the maze facing an open arm. Animals were given a period of 2min to acclimatize with its surroundings. The time spent in each of the two types of arm was recorded during the next 5 min test period. The percentage open arm time were used as index of anxiety. The apparatus was cleaned thoroughly between trials with damp and dry towels.
Diazepam (1.5 mg/kg) and the *Cassia tora* leaves extract (100mg/kg) was orally administered one hour prior to the experiment.

**Locomotor Activity monitor (For positive symptoms):**

Gross open field activity is studied using Digiscan Infrared Photocell system [Test Box model: RXYZCM (16 TAO)]; Omnitech Electronics, Columbus, Ohio] in 42 X 42 X 30 cm Plexiglass arenas, fitted into infrared beam containing metallic grid. Activity of animals will be observed by the interruption of infrared beams. All cages are connected with a counting module, which counts the number of interruptions. Prior to the experiment, animals both treated and the controls, are habituated in the Test Box for 15 minutes. After the initial habituation process, the activity of the treated and control animals is studied for duration of 2 hours (which can vary depending on experimental requirements). Gross locomotor activity of animals is studied in terms of: horizontal activity, total distance traveled (cm), number of movements, movement time (sec), rest time (sec),vertical activity, number of vertical movements, vertical time (sec), stereotypy counts, number of stereotypy, stereotypy time (sec), clockwise revolutions, anti-clockwise revolutions, margin time (sec), centre time (sec), time spent in corners(sec) - Left-Front, Right-Front, Left-Rear, Right-Rear. Data will be  automatically generated through the associated software. Data is presented as Means $\pm$ SEM. Parametric data is analyzed by Student's T test or analysis of variance (ANOVA).
**Rota rod test in mice:** Rota rod test is commonly used for evaluation of neurotoxicity or neurological deficit in mice treated with various plant extracts used in the study. The protocol as described by Dunham and Miya is based on the assumption that an animal with normal motor efficiency (and not reduced muscular tone) is able to maintain its equilibrium on a rotating rod. Basically the Rota rod consists of a rod which is coated with rubber or polypropylene foam to provide friction and to prevent animals from slipping off the rod. The distance between the rod and floor is kept 15cm to avoid intentional jumping of mice. The rod is driven by a motor and the rotational speed can be regulated which is maintained at 80 rpm in our study. Animals were trained on the Rota rod for duration of 2 minutes per trial, with 4 trials per day for two days. On the third day, mice were tested before and one hour after the treatment of extract.
*The following examples are given by way of illustration and should not be construed to limit the scope of the invention.*

**EXAMPLE-1**

The plant material was collected from Bhavnagar city area of Bhavnagar district of Gujarat, located at 12° 75' N Latitude & 72° 15' E Longitude at altitude of 00 m sea level. It was further processed to separate necessary part (leaves) and it was cleaned to remove extraneous matter. The cleaned leaves were air dried at 28°C and powdered in mini grinding mill to obtain powder form of the mesh size of 16$\mu$. The powdered plant material was then further processed for crude extract preparation with solvent system i.e., methanol-water (95:05) at 28°C. The extract was concentrated by conventional techniques using Rotary Evaporator and removed the solvent to the maximum extent. The concentrated extract was freeze dried at the temperature range of -60°C for a period of 16 hours to obtain the crude dried extract. The crude extract was tested for *in vitro* anti-anxiety activity. The activity results are as given below (Table: 1):

**Table 1:** *In vitro* radioligand receptor binding effect of extract on frontocortical benzodiazepine receptors

| Sr.No. | Leaves Extract (Concentration in $\mu$g) | % Inhibition in 3H - Flunitrazepam binding |
|---|---|---|
| 1 | 100 | 86 |
| 2 | 50 | 82 |
| 3 | 25 | 73 |
| 4 | 12.5 | 80 |
| 5 | 6.25 | 69 |
| 6 | 3.12 | 69 |
| 7 | 1.56 | 62 |

## EXAMPLE - 2

The *in-vitro* active extract of Example -1 i.e., the methanol:water :: 95:05 extract of leaves was tested for in-vivo anti-anxiety activity in mice. Rota rod test is commonly used for evaluation of neurotoxicity or neurological deficit in mice treated with various plant extracts used in the study.

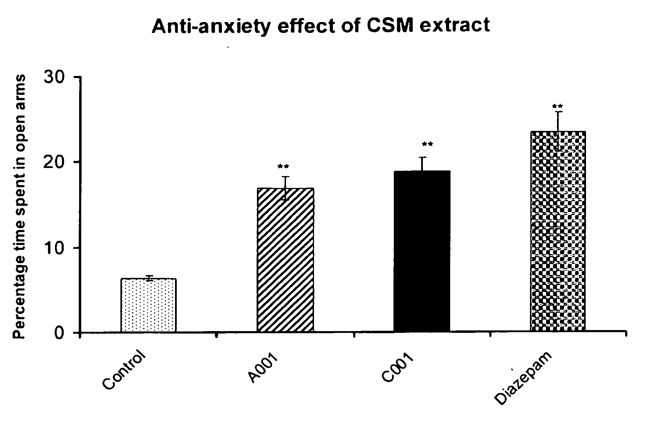

Results:The results are shown in Table 2 and Fig.1. Both original and repeat extract (100 mg/kg p.o.) and Diazepam (1.5 mg/kg p.o.) resulted in a significant increase in the percentage of time spent in open arms ($P < 0.01$) in comparison to saline treated control mice.

**Table 2:**

| DRUG | Dose (mg/kg p.o.) | Time spent in open arms (secs) out of 300 secs* | % time spent in open arms | Significance P value** |
|---|---|---|---|---|
| Control | - | 18.35$\pm$ 0.79 | 6.35 | - |
| Original extract | 100 | 50.57$\pm$4.50 | 16.85 | P<0.01 |
| Repeat extract | 100 | 56.47$\pm$ 6.97 | 18.82 | P<0.01 |
| Diazepam | 1.5 | 70.16$\pm$4.09 | 23.38 | P<0.01 |
| *Values are expressed as Mean$\pm$SEM. **Statistical analysis is done using One way Anova followed by Dunnett's Multiple Comparison Test | | | | |

**Advantages of the invention:**

1. Plant material retains the activity when stored under ambient condition.
2. The extract prepared from the leaves has high shelf life without loss of activity.

**Claims**

1.   A herbal extract of *Cassia tora* leaves prepared by a process comprising the steps of:

i. collecting *Cassia tora* leaves;

ii. drying the leaves at ambient temperature in the range of 25 to 37°C while maintaining the moisture in the range of 0.5- 1.5 %;

iii. pulverizing the leaves dried in step (ii) and selecting the leaves material in the size range of 16 - 20 mesh sieve;

iv. soaking the pulverized leaves obtained in step (iii) above in 95:05 (v/v) alcohol-water mixture in static condition at ambient temperature followed by draining and collecting the extract solution;

v. repeating the soaking step described in step (iv) above five times with fresh solvent mixture at an interval of 24 hours;

vi. concentrating the extract solution obtained in step (v) by known methods;

vii. freeze drying the concentrated extract obtained in step (vi) at a temperature in the range of -50 to -60°C for a period in the range of 8 to 16 hours

for use in treating anxiety disorders.

2. The herbal extract for use according to claim 1, wherein the extract is active on benzodiazepine receptors.

3. The herbal extract for use according to claim 2, wherein the extract inhibits binding of a positive competitor to benzodiazepine receptors even at concentration as low as 1.56 $\mu$g.

4. The herbal extract for use according to any of claims 1 to 3, wherein the extract is administered in a form selected from tablets, lozenges, capsules, powder, solution, intravenously and orally.

**Patentansprüche**

1. Pflanzenextrakt aus Cassia *tora* Blättern, hergestellt durch ein Verfahren, das die folgenden Schritte aufweist:

i. Sammeln der Cassia *tora* Blätter;

ii. Trocknen der Blätter bei Raumtemperatur im Bereich von 25 bis 37°C, wobei gleichzeitig die Luftfeuchte im Bereich von 0,5 - 1,5 % aufrechterhalten wird;

iii. Pulverisieren der in Schritt (ii) getrockneten Blätter und Auslesen des Blattmaterials im Maschensieb-Größenbereich von 16 - 20;

iv. Einweichen der oben in Schritt (iii) erhaltenen pulverisierten Blätter in einer 95:05 (v/v) Alkohol-Wasser Mischung unter statischen Bedingungen bei Raumtemperatur, gefolgt von einem Ablassen und Auffangen der Extraktlösung;

v. Fünfmaliges Wiederholen des oben in Schritt (iv) beschriebenen Einweichungsschritts mit frischer Lösungsmischung in Intervallen von 24 Stunden;

vi. Konzentrieren der in Schritt (v) erhaltenen Extraktlösung mittels bekannten Verfahren;

vii. Gefriertrocknen des in Schritt (vi) erhaltenen konzentrierten Extrakts bei einer Raumtemperatur im Bereich von -50 bis -60°C über einen Zeitraum im Bereich von 8 bis 16 Stunden

zur Verwendung in der Behandlung von Angststörungen.

2. Der Pflanzenextrakt zur Verwendung nach Anspruch 1, wobei der Extrakt aktiv auf Benzodiazepin-Rezeptoren wirkt.

3. Der Pflanzenextrakt zur Verwendung nach Anspruch 2, wobei der Extrakt die Bindung eines positiven Kompetitors an Benzodiazepin-Rezeptoren inhibiert, sogar bei niedrigen Konzentrationen wie 1.56 $\mu$g.

4. Der Pflanzenextrakt zur Verwendung nach einem der Ansprüche 1 bis 3, wobei der Extrakt in einer Form verabreicht wird, die ausgewählt ist aus Tabletten, Lutschtabletten, Kapseln, Pulvern, Lösungen, intravenös und oral.

**Revendications**

1. Extrait d'herbes aromatiques de feuilles de *Cassia tora,* préparé par un procédé comprenant les étapes :

i. de collecte de feuilles de *Cassia tora* ;

ii. de séchage des feuilles à température ambiante dans la plage de 25 à 37°C, tout en maintenant une humidité dans la plage de 0,5-1,5% ;

iii. de pulvérisation des feuilles séchées dans l'étape (ii) et de sélection des matériaux foliaires dans la plage de taille de tamis de 16-20 mesh ;

iv. de trempage des feuilles pulvérisées obtenues dans l'étape (iii) ci-dessus dans un mélange 95:05 (v/v) d'alcool-eau en conditions statiques à température ambiante, suivi d'un égouttage et d'une collecte de la solution d'extrait ;

v. de répétition de l'étape de trempage décrite dans l'étape (iv) ci-dessus cinq fois avec un mélange de solvants frais à un intervalle de 24 heures ;

vi. de concentration de la solution d'extrait obtenue à l'étape (v) par des méthodes connues ;

vii. de lyophilisation de l'extrait concentré obtenu à l'étape (vi) à une température dans la plage allant de -50 à -60°C pendant une période dans la plage allant de 8 à 16 heures ;

pour une utilisation pour le traitement de troubles de l'anxiété.

2. Extrait d'herbes aromatiques pour une utilisation selon la revendication 1, **caractérisé en ce que** l'extrait est actif sur les récepteurs de benzodiazépine.

3. Extrait d'herbes aromatiques pour une utilisation selon la revendication 2, **caractérisé en ce que** l'extrait inhibe la fixation d'un compétiteur positif aux récepteurs de benzodiazépine même à une concentration aussi faible que 1,56 μg.

4. Extrait d'herbes aromatiques pour une utilisation selon l'une quelconque des revendications 1 à 3, **caractérisé en ce que** l'extrait est administré sous une forme choisie parmi les comprimés, les tablettes, les capsules, les poudres, les solutions, par voie intraveineuse et orale.

**Anti-anxiety effect of CSM extract**

**EP 2 411 030 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008015697 A3
- WO 2008020311 A3
- WO 2008032958 A1
- CN 101167908 A
- CN 101167954 A
- CN 101120708 A
- CN 101112457 A
- CN 101112432 A
- CN 101112468 A
- JP 2008007473 A
- US 20070014749 A1
- WO 2007004570A1 PCT

- WO 2007111401 A1
- WO 2006081739 A1
- KR 2006030535 A
- KR 646594 B1
- WO 2004112733A1 A
- US 20080004340 A1
- US 7262157 B2
- WO 03105599 A1
- EP 01554938 B1
- EP 01554938 A
- US 4753659 A
- KR 712055 B1

### Non-patent literature cited in the description

- Mental Disorders. **REUS V.I.** Harrison's Principles of internal medicine. 2005, 2457-2552
- Mental Disorders. **REUS V.I.** Harrison's Principles of internal medicine. Mc-Graw Hill, 2005, 2457-2552
- Hypnotics and Sedatives. **CHARNEY D.S. ; MIHIC S.J. ; HARRIS R.A.** Goodmann and Gillmann's the pharmacological basis of therapeutics. 2006, 401-427
- Drug therapy of Depression and Anxiety Disorders. **MC-GRAW HILL ; BALDESSARINI R.J.** Goodmann and Gillmann's the pharmacological basis of therapeutics. Mc-Graw Hill, 2006, 429-459
- Hypnotics and Sedatives. **CHARNEY D.S. ; MIHIC S.J. ; HARRIS R.A.** Goodmann and Gillmann's the pharmacological basis of therapeutics. Mc-Graw Hill, 2006, 401-427
- **WOOD, J.H. ; KATZ, J.L ; WINGER G.** Benzodiazepines: Use,abuse, and consequences. *Pharmacol. Rev.,* 1992, vol. 44, 151-347
- **PETURSSON, H.** The benzodiazepine withdrawal syndrome. *Addiction,* 1994, vol. 89, 1455-1459
- Drug therapy of Depression and Anxiety Disorders. **BALDESSARINI R.J.** Goodmann and Gillmann's the pharmacological basis of therapeutics. Mc-Graw Hill, 2006, 429-459
- Hypnotics and Sedatives. **CHARNEY D.S. ; MIHIC S.J. ; HARRIS R.A.** Goodmann and Gilimann's the pharmacological basis of therapeutics. Mc-Graw Hill, 2006, 401-427

- Anthraquinones of edible wild vegetable Cassia tora stimulate proliferation of human CD4(+) T lymphocytes and secretion of interferon - gamma or interleukin 10. *Food Chemistry,* April 2008, vol. 107 (4), 1576-1580
- **ZHANG, Y et al.** Chemical components and antioxidant activity of the volatile oil from cassia tora L. seed prepared by supercritical fluid extraction. *Journal Of Food Lipids,* December 2007, vol. 14 (4), 411-423
- **JANG DS et al.** Anthraquinones from the seeds of Cassia tora with inhibitory activity on protein glycation and aldose reductase. *Biological & Pharmaceutical Bulletin,* November 2007, vol. 30 (11), 2207-2210
- **EL-HALAWANY, AM et al.** Estrogenic and anti-estrogenic activities of cassia tora phenolic constituents. *Chemical & Pharmaceutical Bulletin,* October 2007, vol. 55 (10), 1476-1482
- **JIA, ZB et al.** Antioxidant properties of extracts from juemingzi (Cassia tora L.) evaluated in vitro. *LWT-Food Science And Technology,* 2007, vol. 40 (06), 1072-1077
- **CHO, IJ et al.** Hypolipidemic effect of soluble fiber isolated from seeds of Cassia tora Linn in rats fed a high-cholesterol diet. *Journal Of Agricultural And Food Chemistry,* 21 February 2007, vol. 55 (04), 1592-1596
- Identification of antifungal activity in different species of Cassia. *Indian Journal of Environment and Eco-planning,* 2006, vol. 12 (3), 661-663

18

- Effects of Cassia tora fiber supplement on serum lipids in Korean diabetic patients. *Journal Of Medicinal Food,* 2005, vol. 8 (3), 31 1-318
- **LEE,CH ; LEE, HS.** antifungal property of dihydroxy-anthraquinones against phytopathogenic fungi. *Journal Of Microbiology And Biotechnology,* April 2005, vol. 15 (2), 442-446
- **CHI-HAO WU ; GOW-CHIN YEN.** Antigenotoxic properties of Cassia tea (Cassia tora L.): Mechanism of action and the influence of roasting process. *Life Sciences,* November 2004, vol. 76 (1), 85-101
- **KIM, YM et al.** Anthraquinones isolated from Cassia tora (Leguminosae) seed show an antifungal property against phytopathogenic fungi. *Journal Of Agricultural And Food Chemistry,* 06 October 2004, vol. 52 (20), 6096-6100
- **HEBBAR, SS et al.** Ethnomedicine of Dharwad district in Karnataka, India - plants used in oral health care. *Journal Of Ethnopharmacology,* October 2004, vol. 94 (2-3), 261-266
- **PARK, TH et al.** Peroxynitrite scavenging mode of alaternin isolated from Cassia tora. *Journal Of Pharmacy And Pharmacology,* October 2004, vol. 56 (10), 1315-1321
- **PATIL, UK et al.** Hypolipidemic activity of seeds of Cassia tora Linn. *Journal Of Ethnopharmacology,* February 2004, vol. 90 (2-3), 249-252
- **LEE, HS.** Inhibitory effects of quinizarin isolated from Cassia tora seeds against human intestinal bacteria and Aflatoxin B-1 biotransformation. *Journal Of Microbiology And Biotechnology,* August 2003, vol. 13 (4), 529-536
- **CHIDUME, FC et al.** Antinociceptive and smooth muscle contracting activities of the methanolic extract of Cassia tora leaf. *Journal Of Ethnopharmacology,* July 2002, vol. 81 (2), 205-209
- **YEN, GC et al.** Antioxidant properties of water extracts from Cassia tora L. in relation to the degree of roasting. *Journal Of Agricultural And Food Chemistry,* July 2000, vol. 48 (7), 2760-2765
- **EL-TAHIR, A et al.** Antiplasmodial activity of selected sudanese medicinal plants with emphasis on Acacia nilotica. *Phytotherapy Research,* September 1999, vol. 13 (6), 474-478
- **HATANO, T et al.** Phenolic constituents of cassia seeds and antibacterial effect of some naphthalenes and anthraquinones on methicillin-resistant Staphylococcus aureus. *Chemical & Pharmaceutical Bulletin,* August 1999, vol. 47 (8), 1121-1127
- **AGARKAR, SV et al.** Photochemical and pharmaco-logical investigations of genus Cassia: A review. *Asial Journal Of Chemistry,* April 1999, vol. 11 (2), 295-299
- **MAITY, TK et al.** Studies on antiinflammatory effect of Casia tora leaf extract (fam. Leguminosae. *Phytotherapy Research,* May 1998, vol. 12 (3), 221-223
- **YEN, GC et al.** Extraction and identification of an antioxidative component from Jue Ming Zi (Cassia tora L. *Journal Of Agricultural And Food Chemistry,* March 1998, vol. 46 (3), 820-824
- **PANDEY, VN et al.** Synergistic activity of extracted plant oils against Pythium aphanidermatum and P-debaryanum. *Tropical Agriculture,* April 1997, vol. 74 (2), 164-167
- **CHOI, JS et al.** In vitro antimutagenic effects of anthraquinone aglycones and naphthopyrone glycosides from Cassia tora. *Planta Medica,* February 1997, vol. 63 (1), 11-14
- **MUKHERJEE, PK et al.** Antifungal activities of the leaf extract of Cassia tora Linn (Fam Leguminosae. *Phytotherapy Research,* September 1996, vol. 10 (6), 521-522
- **SUI-MING WONG et al.** New Antihepatotoxic Naphthopyrone Glycosides from the Seeds of Cassia tora. *Planta Medica,* June 1989, vol. 55 (3), 276-280
- **SUI-MING WONG et al.** Anthraquinone glycosides from the seeds of Cassia tora. *Phytochemistry,* 1989, vol. 28 (1), 211-214